(19) Europäisches Patentamt
European Patent Office
Office européen des brevets

(11) Veröffentlichungsnummer: **0 221 023 A2**

(12) **EUROPÄISCHE PATENTANMELDUNG**

(21) Anmeldenummer: **86810466.2**

(22) Anmeldetag: **20.10.86**

(51) Int. Cl.⁴: **C 07 D 213/84**, C 07 D 213/79, C 07 D 217/26 // C07D211/98

(30) Priorität: **25.10.85 CH 4609/85**

(43) Veröffentlichungstag der Anmeldung: **06.05.87 Patentblatt 87/19**

(84) Benannte Vertragsstaaten: **AT BE CH DE ES FR GB IT LI NL SE**

(71) Anmelder: **CIBA-GEIGY AG, Klybeckstrasse 141, CH-4002 Basel (CH)**

(72) Erfinder: **Waldner, Adrian, Dr., Holeeweg 39, CH-4123 Allschwil (CH)**

(54) **Verfahren zur Herstellung von Pyridin-2-carbonsäurederivaten und 1-Amino-1,4-dihydropyridin-2-carbonsäurederivate.**

(57) Das erfindungsgemässe Verfahren betrifft die Umsetzung von Hydrazonen der Formel II

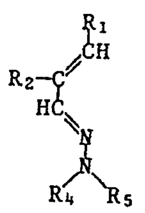

(II)

mit α-Chlor- oder α-Bromacrylnitril oder -acrylsäureestern der Formel III

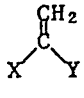

(III)

zu 1-Amino-2-cyano- oder 1-Amino-2-carbonsäureester-1,4-dihydropyridinen der Formel IV

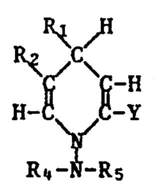

(IV),

welche durch Behandlung mit Säuren unter Abspaltung von $R_4R_5NH$ in Pyridinderivate der Formel I

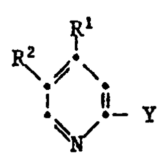

(I),

übergeführt werden. Y ist hierin $-CN$ oder $-COOR_3$. $R_1$ bis $R_5$ sind z.B. Alkyl.

EP 0 221 023 A2

CIBA-GEIGY AG
Basel (Schweiz)

5-15577/+/ZFO

Verfahren zur Herstellung von Pyridin-2-carbonsäurederivaten und 1-Amino-1,4-dihydropyridin-2-carbonsäurederivate

Die vorliegende Erfindung betrifft eine Diels-Alder-Reaktion, bei der ein ungesättigtes Hydrazon mit α-Halogenacrylnitril oder α-Halogenacrylsäureester zu einem 1-Amino-2-cyano- oder 1-Amino-2-carbonsäureester-1,4-dihydropyridin umgesetzt wird, das durch Behandlung mit einer Säure in die entsprechenden Pyridinderivate übergeführt wird.

In Tetrahedron Letters, Vol. 23, No. 32, pp 3261-3264 (1982) ist die Diels-Alder-Reaktion von 1-Amino-1-aza-3-methyl-1,3-butadien mit Acrylnitril bzw. Methylacrylat unter Bildung von 1-Amino-3-methyl-2-cyano- bzw.- 2-carbomethoxy-1,2,3,4-tetrahydropyridin beschrieben. Die Abspaltung der Aminogruppe unter reduktiven Bedingungen führt zu Piperidinderivaten, aber nicht zu Pyridinderivaten. Ziel vorliegender Erfindung ist es, ein Verfahren bereitzustellen, mit dem regiospezifisch substituierte 2-Cyano- oder 2-Carbonsäureesterpyridine aus billigen und leicht zugänglichen Ausgangsstoffen in guten Ausbeuten hergestellt werden können.

Gegenstand der Erfindung ist ein Verfahren zur Herstellung von 2-Cyanopyridinen oder Pyridin-2-carbonsäureestern der Formel I

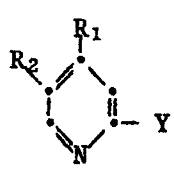

(I),

worin $R_1$ Wasserstoff; eine unsubstituierte oder substituierte, lineare oder verzweigte Alkyl-, Alkylthio- oder Alkoxygruppe; eine unsubstituierte oder substituierte Phenyl- oder Phenoxygruppe bedeutet; $R_2$ unabhängig die gleiche Bedeutung hat wie $R_1$ und zusätzlich Fluor, Chlor oder Brom bedeutet; oder $R_1$ und $R_2$ gemeinsam für eine substituierte oder unsubstituierte $C_3$-$C_4$-Alkylenbrücke stehen und Y CN oder $COOR_3$ ist, worin $R_3$ Alkyl, Cycloalkyl, Aryl oder Aralkyl darstellt, dadurch gekennzeichnet, dass man

(a) ein Hydrazon der Formel II

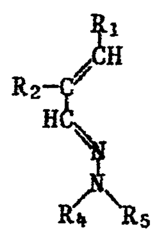

(II),

in welcher $R_1$ und $R_2$ die für Formel I angegebene Bedeutung haben und $R_4$ und $R_5$ einzeln je Alkyl, Cycloalkyl, Aralkyl oder Aryl oder zusammen Alkylen oder Oxaalkylen bedeuten, in Gegenwart eines inerten Lösungsmittels und einer Base mit α-Chlor- oder α-Brom-acrylnitril oder -acrylsäureester der Formel III

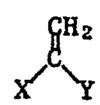

(III) ,

worin X für Halogen steht und Y die in Formel I angegebene Bedeutung hat, zu einem 1-Amino-2-cyano- oder 1-Amino-2-carbonsäureester-1,4-dihydropyridin der Formel IV

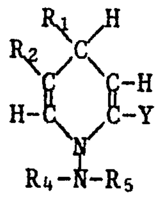

(IV),

worin $R_1$, $R_2$, $R_4$, $R_5$ und Y die zuvor angegebene Bedeutung haben, umsetzt, und

(b) das 1-Amino-2-cyano- oder 1-Amino-2-carbonsäureester-1,4-dihydropyridin der Formel IV durch Behandlung mit einer Säure unter Abspaltung von $HNR_4R_5$ in ein 2-Cyanopyridin oder einen Pyridin-2-carbonsäureester der Formel I überführt.

Halogen ist Fluor, Chlor, Brom oder Jod.

X in Formel II steht bevorzugt für Cl oder Br.

In obigen Formeln enthalten $R_1$ und $R_2$ als Alkyl, Alkylthio und Alkoxy bevorzugt 1 bis 6 C-Atome. Geeignete Substituenten für diese Reste sind zum Beispiel Halogen, besonders Fluor, Chlor und Brom, $C_1$-$C_4$-Alkoxy, Phenyl, Phenoxy, Cyano und $C_1$-$C_4$-Alkoxycarbonyl. Bevorzugte Substituenten sind Halogen. Beispiele für diese Reste sind: Methyl, Ethyl, n-Propyl, i-Propyl, n-Butyl, i-Butyl, t-Butyl, 1-Pentyl, 3-Pentyl, 1-Hexyl, 2-Hexyl, 3-Hexyl, Methoxy, Ethoxy, n-Propoxy, i-Propoxy, n-Butoxy, i-Butoxy, t-Butoxy, Methylthio, Ethylthio, Propylthio, Butylthio, Hydroxymethyl, Fluormethyl, Trifluormethyl, 2-Cyanoethyl, 2-Chlorethyl, Brommethyl, Benzyl, Chlorbenzyl, Methoxymethyl, Ethoxymethyl, Methoxyethyl.

Geeignete Substituenten für $R_1$ und $R_2$ als Phenyl und Phenoxy sind z.B. Halogen, wie F, Cl und Br, CN, $C_1$-$C_4$-Alkyl, $C_1$-$C_4$-Alkoxy, $C_1$-$C_4$-Haloalkyl oder $C_1$-$C_4$-Cyanoalkyl. Beispiele sind Methylphenyl, Ethylphenyl, Methoxyphenyl, Ethoxyphenyl, Chlorphenyl, Fluorphenyl, Difluorphenyl, Bromphenyl, Chlorphenoxy, Methylphenoxy, Methoxyphenoxy, Fluormethylphenyl, Difluormethylphenyl, Trifluormethylphenyl, Chlormethylphenyl, Cyanomethylphenyl, 2-Cyanoethylphenyl, Trifluormethylphenoxy und Cyanomethylphenoxy.

In einer bevorzugten Ausführungsform ist $R_1$ Wasserstoff. $R_2$ ist bevorzugt $C_1$-$C_6$-Alkyl, $C_1$-$C_6$-Alkoxy oder $C_1$-$C_6$-Alkylthio, wobei der Alkylteil ein oder mehrfach halogensubstituiert sein kann.

$R_3$ kann als Alkyl linear oder verzweigt sein und bevorzugt 1 bis 6 C-Atome enthalten. $R_3$ ist als Cycloalkyl bevorzugt Cyclopentyl oder Cyclohexyl. $R_3$ als Aryl bzw. Aralkyl ist bevorzugt Phenyl oder Benzyl. Das Phenyl oder Benzyl kann substituiert sein z.B. durch $C_1$-$C_4$-Alkyl, $C_1$-$C_4$-Alkoxy oder Halogen, wie Fluor, Chlor oder Brom.

$R_4$ und $R_5$ als Alkyl können linear oder verzweigt sein und enthalten bevorzugt 1 bis 6, besonders 1 bis 4 C-Atome. Besonders bevorzugte Reste $R_4$ und $R_5$ sind Ethyl oder Methyl. Als Cycloalkyl sind $R_4$ und $R_5$ bevorzugt Cyclopentyl oder Cyclohexyl, als Aryl bevorzugt Phenyl und als Aralkyl bevorzugt Phenylalkyl mit 1 bis 4 C-Atomen in der Alkylgruppe, besonders Benzyl.

$R_4$ und $R_5$ zusammen sind als Alkylen bevorzugt Tetramethylen oder Pentamethylen und als Oxaalkylen 3-Oxapentylen.

$R_1$ und $R_2$ sind zusammen als $C_3$-$C_4$-Alkylen bevorzugt Tetramethylen. Die so gebildete Trimethylen- bzw. Tetramethylengruppe kann mehrfach durch Halogen, Alkoxy, Alkylthio, Cyano, Halogenalkyl, Halogenalkoxy oder Halogenalkylthio substituiert sein.

Geeignete Lösungsmittel zur Durchführung des erfindungsgemässen Verfahrens [Reaktionsstufe (a)] sind solche, die gegenüber den Reaktionsteilnehmern inert sind, z.B. polare, aprotische Lösungsmittel, die einzeln oder in Mischungen aus mindestens zwei Lösungsmitteln verwendet werden können. Beispiele sind: Ether, wie Dibutylether, Tetrahydrofuran, Dioxan, Dimethylethylenglykol, Diethyldiethylenglykol, Dimethyltriethylenglykol, halogenierte Kohlenwasserstoffe wie Methylenchlorid, Chloroform, 1,2-Dichlorethan, 1,1,1-Trichlorethan, 1,1,2,2-Tetrachlorethan, Carbonsäureester und Lactone wie Essigsäureethylester, Propionsäuremethylester, Benzoesäureethylester, 2-Methoxyethylacetat, γ-Butyrolacton, o-Valerolacton und Pivalolacton, Carbonsäureamide und Lactame, wie Formamid, Acetamid, N-Methylformamid, N,N-Dimethylformamid, N,N-Diethylformamid, N,N-Dimethylacetamid, N,N-Diethylacetamid, γ-Butyrolactam, ξ-Caprolactam, N-Methylpyrrolidon, N-Acetylpyrrolidon, N-Methylcaprolactam, Tetramethylharnstoff, Hexamethylphosphorsäuretriamid, Sulfoxide, wie Dimethylsulfoxid, Sulfone, wie Dimethylsulfon, Diethylsulfon, Trimethylensulfon, Tetramethylensulfon, tertiäre Amine wie Trimethylamin, Triethylamin, N-Methylpyrrolidin, N-Methylpiperidin, N-Methylmorpholin, substituierte Benzole, wie Chlorbenzol, Nitrobenzol, Nitrile, wie z.B. Acetonitril. Solche Lösungsmittel werden vorteilhaft auch in der Reaktionsstufe (b) verwendet.

Die Verbindungen der Formeln II und III sind bekannt oder können nach bekannten Verfahren hergestellt werden. Sie werden bevorzugt in äquimolaren Mengen eingesetzt. Es kann aber auch ein geringer Ueberschuss der ungesättigten Hydrazone der Formel II verwendet werden.

Die Reaktionstemperatur beträgt in der ersten Stufe bevorzugt mindestens 40°C, insbesondere 40 bis 100°C. Als Basen eignen sich besonders im verwendeten Lösungsmittel lösliche Basen. Geeignete Basen sind z.B. Alkalimetallcarbonate, wie Lithium-, Natrium- oder Kaliumcarbonat, und besonders organische Amine, insbesondere tertiäre Amine, die gleichzeitig als Lösungsmittel dienen können. Bevorzugt sind tertiäre Amine mit $C_1$-$C_4$-Alkylresten, wie z.B. Triethylamin.

In einer vorteilhaften Ausführungsform werden das Hydrazon der Formel II und die Base im Lösungsmittel gelöst, und langsam zu der erwärmten Lösung die Verbindung der Formel III zugegeben.

Die 1-Amino-1,4-dihydropyridin-derivate der Formel IV können als Rohprodukt eingesetzt werden oder vor der zweiten Reaktionsstufe isoliert werden. Bei der Durchführung der Reaktionsstufe (a) kann bereits teilweise die gewünschte Verbindung der Formel I gebildet werden. Die Behandlung mit einer Säure wird bevorzugt bei Raumtemperatur vorgenommen. Geeignete Säuren sind besonders Mineralsäuren, z.B. Halogenwasserstoffsäuren (Salzsäure, Bromwasserstoffsäure, Jodwasserstoffsäure), Perchlorsäure oder Schwefelsäure. Bevorzugt setzt man in einem organischen Lösungsmittel gelösten Chlorwasserstoff, Bromwasserstoff oder Jodwasserstoff ein, besonders Chlorwasserstoff.

Die Isolierung und Reinigung der erfindungsgemäss hergestellten Verbindungen erfolgt nach üblichen Methoden durch Filtration, Extraktion, Destillation, Kristallisation oder chromatographische Methoden.

Ein weiterer Gegenstand der Erfindung sind 1-Amino-2-cyanodihydropyridine und 1-Amino-dihydropyridin-2-carbonsäureester der Formel IV

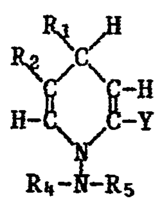

(IV),

worin $R_1$ Wasserstoff; eine unsubstituierte oder substituierte, lineare oder verzweigte Alkyl-, Alkylthio- oder Alkoxygruppe; eine substituierte oder unsubstituierte Phenyl- oder Phenoxygruppe bedeutet; $R_2$ unabhängig die gleiche Bedeutung hat wie $R_1$ und zusätzlich Fluor, Chlor oder Brom bedeutet; oder $R_1$ und $R_2$ gemeinsam für eine substituierte oder unsubstituierte $C_3$-$C_4$-Alkylenbrücke stehen und Y CN oder $COOR_3$ ist, worin $R_3$ Alkyl, Cycloalkyl, Aryl oder Aralkyl darstellt, und $R_4$ und $R_5$ einzeln je Alkyl, Cycloalkyl,

Aralkyl oder Aryl oder zusammen Alkylen oder Oxaalkylen bedeuten. Für $R_1$, $R_2$, $R_3$, $R_4$, $R_5$ und Y gelten die zuvor erwähnten Bevorzugungen.

Mit dem erfindungemässen Verfahren werden die Pyridinderivate der Formel I in hohen Ausbeuten aus einfach zugänglichen und billigen Ausgangsmaterialien erhalten. Die Synthese ist einfach durchführbar und regiospezifisch.

Die Verbindungen der Formel I können z.B. als Pharmazeutika verwendet werden, wie etwa Fusarsäure, [vgl. Pharmazie 39, Heft 3, S. 155 (1984)]. Sie sind ferner wertvolle Zwischenprodukte für Agrowirkstoffe, z.B. nach Substitution der Nitrilgruppe (via Amid, Amin) durch eine Halogengruppe.

So können beispielsweise die aus der europäischen Patentanmeldung EP-A 0 185 621 bekannten 2-Chlor-5-halogenalkylpyridine, wie etwa das 2-Chlor-5-pentachlorethylpyridin VIII ausgehend von 2-Cyano-5-ethylpyridin der Formel I ($R_1$ = H, $R_2$ = $C_2H_5$, Y =CN) gemäss nachstehendem Reaktionsschema

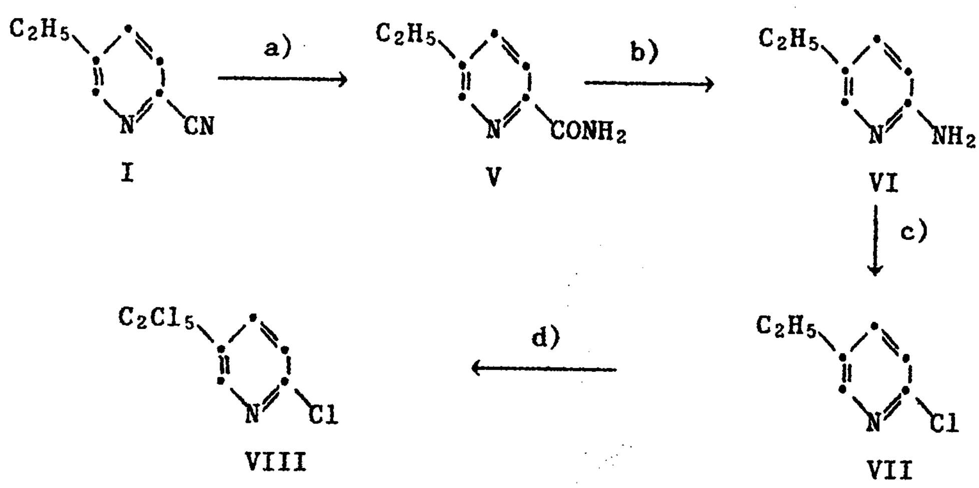

I
V
VI
VII
VIII

hergestellt werden.

Die einzelnen Verfahrensstufen a) bis d) können analog literaturbekannter Verfahren durchgeführt werden. (Houben-Weyl, Methoden der Organischen Chemie, Thieme, Stuttgart 1952, Bd 8, S. 663; L.N. Yakhoutov et al., Khim. Geterotsikl-Soedin 1967 1063-7; EP-A 0 186 621).

Die nachfolgenden Beispiele erläutern die Erfindung.

Beispiel 1: Herstellung von 5-Ethyl-2-cyan-pyridin

39,3 g 1-Dimethylamino-1-aza-3-ethyl-1,3-butadien, 35 g 2-Chloracrylnitril und 112 ml Triethylamin werden in 400 ml Acetonitril 7 Stunden bei 70°C gerührt. Nach Abkühlen der braunen Reaktionsmischung wird filtriert und eingedampft. Der Rückstand wird in Dioxan gelöst und gasförmiges HCl eingeleitet. Nach 1 h Rühren bei Raumtemperatur (RT) wird eingedampft und zwischen Chloroform und 2n Sodalösung verteilt. Die organische Phase wird abgetrennt, getrocknet und eingedampft. Der ölige Rückstand wird am Hochvakuum (HV) destilliert. Man erhält 30,6 g leicht gelbes Oel vom Siedepunkt (Sdp.) 75-79°C/$3 \cdot 10^{-1}$mbar.

IR ($CHCl_3$) : 2240 $cm^{-1}$ (C≡N)

Beispiel 2: Herstellung von 5-n-Butyl-2-cyan-pyridin

Je 0,1 Mol 1-Dimethylamino-1-aza-3-n-butyl-1,3-butadien und 2-Chloracrylnitril werden mit 0,2 Mol Triethylamin in 100 ml Acetonitril 20 h auf 60°C erwärmt. Nach Eindampfen des Reaktionsgemisches wird in 200 ml Dioxan gelöst, filtriert und HCl-Gas eingeleitet. Nach 15 Minuten Rühren bei RT wird eingedampft und zwischen $CH_2Cl_2$ und 1n $NaHCO_3$ verteilt. Die organische Phase liefert nach Trocknen, Eindampfen und Hochvakuumdestillation 10,0 g (63 %) eines leicht gelben Oeles. Sdp. 86-87°C/$3 \cdot 10^{-1}$ mbar.

Beispiel 3: Herstellung von 4,5-Dimethyl-2-cyan-pyridin

Die Durchführung erfolgt analog Beispiel 2 mit 1-Dimethylamino-1-aza-3,4-dimethyl-1,3-butadien. Der Rückstand wird HV-destilliert (Sdp. 91°C). Man erhält leicht gelbe Kristalle vom Schmelzpunkt (Smp.) 72-74°C (Digerieren in Ether/Hexan (farblose Kristalle).

Beispiel 4: Herstellung von 5-Isopropyl-picolinsäurenitril

83,8 g (0,6 Mol) 1-Dimethylamino-1-aza-3-isopropyl-1,3-butadien und 121.4 g (1,2 Mol) Triethylamin werden in 325 ml Acetontril gelöst. Dazu gibt man 52,5 g (0,6 Mol) α-Chloracrylnitril. Diese Lösung wird 4 h auf 70°C erhitzt, nach Abkühlen filtriert und eingedampft. Der ölige Rückstand wird in Dioxan gelöst und unter Eiskühlung mit gasförmigem HCl versetzt, bis pH 1 erreicht ist. Nach 30 Minuten wird eingedampft und zwischen Chloroform und 1 n $NaHCO_3$ verteilt. Nach Trocknen und Eindampfen liefert die organische Phase ein Oel, das am Hochvakuum bei 81-84°C destilliert. Das Destillat erstarrt und ergibt 60,1 g (68,5 %) Produkt vom Smp. 47-49°C.

Beispiel 5: Herstellung von 2-Cyan-5-methylpyridin

19,6 g 1-Dimethylamino-1-aza-3-methyl-1,3-butadien, 17,5 g 2-Chloracrylnitril und 33,6 g Diazabicyclooctan (DABCO) werden in 200 ml Acetonitril 30 Std. auf Rückfluss erhitzt. Nach Eindampfen wird mit 1N-HCl leicht sauer gestellt und mit Chloroform extrahiert. Nach Trocknen und Eindampfen wird der Rückstand der organischen Phase destilliert. Sdp. ($6 \cdot 10^{-3}$ bar) 63-69°. Das Destillat wird mit Petroläther digeriert.

Man isoliert die Titelverbindung als Kristalle vom Smp. 72-74°.

Beispiel 6: Herstellung von 5-Methylpyridin-2-carbonsäureethylester

13 g 2,3-Dibrompropionsäureethylester werden in 50 ml Dioxan bei 0° mit 6,1 g Triethylamin versetzt. Nach 2 Std. Ausrühren bei RT wird eine Lösung von 5,6 g 1-Dimethylamino-1-aza-3-methyl-1,3-butadien und 6,1 g Triethylamin in 50 ml zugetropft. Nach 30 Std. bei 70° wird abgekühlt, zwischen Wasser und Ether verteilt. Die organische Phase wird mit Sole gewaschen, getrocknet und eingedampft. Der Rückstand liefert nach Destillation unter vermindertem Druck 3,2 g der Titelverbindung als Oel mit Kp = 69-74° (bei 0,08 mbar).

Beispiel 7: Herstellung von 3-Cyano-5,6,7,8-tetrahydroisochinolin

7,6 g Cyclohex-1-en-carboxaldehyd-N,N-dimethylhydrazon, 4,4 g 2-Chloracrylnitril und 10,1 g Triethylamin werden in 75 ml Dioxan 18 h auf 90° erhitzt. Nach Eindampfen wird mit 1n HCl sauer gestellt und 3x mit Chloroform extrahiert. Nach Trocknen und Eindampfen wird an Kieselgel chromatographiert.

Man isoliert die Titelverbindung als schwach gelbe Kristalle vom Smp. 56-59°.

Beispiel 8: Herstellung von 2-Cyano-1,4-dihydro-1-dimethylamino-5-ethyl-pyridin

12,6 g 1-Dimethylamino-1-aza-3-ethyl-1,3-butadien, 8 ml 2-Chloracrylnitril und 27,8 ml Triethylamin werden in 40 ml Dioxan 3,5 h auf 70° erhitzt. Nach Eindampfen wird an Kieselgel mit Hexan/-Essigester 16:1 chromatographiert. Man isoliert 8,2 g der Titelverbindung als gelbes Oel, welches bei Stehenlassen bei Raumtemperatur unter Abspaltung von Dimethylamin spontan zu 2-Cyano-5-ethylpyridin weiterreagiert.

Patentansprüche:

1. Verfahren zur Herstellung von 2-Cyanopyridinen oder Pyridin-2-carbonsäureestern der Formel I

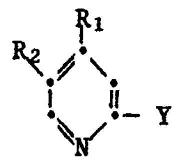

(I),

worin $R_1$ Wasserstoff; eine unsubstituierte oder substituierte, lineare oder verzweigte Alkyl-, Alkylthio- oder Alkoxygruppe; eine unsubstituierte oder substituierte Phenyl- oder Phenoxygruppe bedeutet; $R_2$ unabhängig die gleiche Bedeutung hat wie $R_1$ und zusätzlich Fluor, Chlor oder Brom bedeutet; oder $R_1$ und $R_2$ gemeinsam für eine substituierte oder unsubstituierte $C_3$-$C_4$-Alkylenbrücke stehen; und Y CN oder $COOR_3$ ist, worin $R_3$ Alkyl, Cycloalkyl, Aryl oder Aralkyl darstellt, das dadurch gekennzeichnet ist, dass man

(a) ein Hydrazon der Formel II

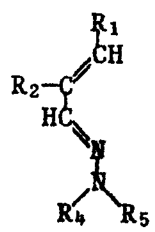

(II),

in welcher $R_1$ und $R_2$ die für Formel I angegebene Bedeutung haben und $R_4$ und $R_5$ einzeln je Alkyl, Cycloalkyl, Aralkyl oder Aryl oder zusammen Alkylen oder Oxaalkylen bedeuten, in Gegenwart eines inerten Lösungsmittels und einer Base mit α-Chlor- oder α-Bromacrylnitril oder -acrylsäureester der Formel III

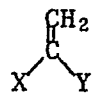

(III),

worin X für Halogen steht und Y die in Formel I angegebene Bedeutung hat, zu einem 1-Amino-2-cyano- oder 1-Amino-2-carbonsäureester-1,4-dihydropyridin der Formel IV

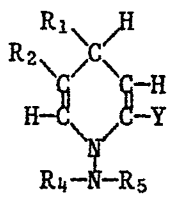

(IV),

worin $R_1$, $R_2$, $R_4$, $R_5$ und Y die zuvor angegebene Bedeutung haben, umsetzt, und

(b) das 1-Amino-2-cyano- oder 1-Amino-2-carbonsäureester-1,4-dihydropyridin der Formel IV durch Behandlung mit einer Säure unter Abspaltung von $HNR_4R_5$ in ein 2-Cyanopyridin oder einen Pyridin-2-carbonsäureester der Formel I überführt.

2. Verfahren gemäss Anspruch 1, dadurch gekennzeichnet, dass $R_1$ und $R_2$ als Alkyl, Alkylthio und Alkoxy 1 bis 6 C-Atome enthalten.

3. Verfahren gemäss Anspurch 1, dadurch gekennzeichnet, dass $R_3$ lineares oder verzweigtes Alkyl mit 1 bis 6 C-Atomen, Cyclopentyl, Cyclohexyl, Phenyl oder Benzyl ist.

4. Verfahren gemäss Anspruch 1, dadurch gekennzeichnet, dass $R_1$ Wasserstoff ist.

5. Verfahren gemäss Anspruch 1, dadurch gekennzeichnet, dass $R_2$ $C_1$-$C_6$-Alkyl ist.

6. Verfahren gemäss Anspruch 1, dadurch gekennzeichnet, dass $R_4$ und $R_5$ unabhängig voneinander lineares oder verzweigtes Alkyl mit 1 bis 6 C-Atomen, Cyclopentyl, Cyclohexyl, Phenyl oder Phenylalkyl mit 1 bis 4 C-Atomen in der Alkylgruppe, oder $R_4$ und $R_5$ zusammen Tetramethylen, Pentamethylen oder 3-Oxapentylen sind.

7. Verfahren gemäss Anspruch 1, dadurch gekennzeichnet, dass in der Reaktionsstufe (a) ein polares aprotisches Lösungsmittel verwendet wird.

8. Verfahren gemäss Anspruch 1, dadurch gekennzeichnet, dass die Reaktionstemperatur in der Reaktionsstufe (a) mindestens 40°C beträgt.

9. Verfahren gemäss Anspruch 1, dadurch gekennzeichnet, dass man in der Reaktionsstufe (a) eine im Lösungsmittel lösliche Base verwendet.

10. Verfahren gemäss Anspruch 9, dadurch gekennzeichnet, dass die Base ein tertiäres Amin ist.

11. Verfahren gemäss Anspruch 1, dadurch gekennzeichnet, dass man die Reaktionsstufe (b) in Gegenwart eines Lösungsmittels bei Raumtemperatur durchgeführt.

12. Verfahren gemäss Anspruch 1, dadurch gekennzeichnet, dass man in Reaktionsstufe (b) als Säure Halogenwasserstoff einsetzt.

13. 1-Amino-2-cyanodihydropyridine und 1-Amino-dihydropyridin-2-carbonsäureester der Formel IV

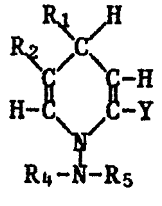

(IV),

worin $R_1$ Wasserstoff; eine unsubstituierte oder substituierte, lineare oder verzweigte Alkyl-, Alkylthio- oder Alkoxygruppe; oder eine substituierte oder unsubstituierte Phenyl- oder Phenoxygruppe bedeutet; $R_2$ unabhängig die gleiche Bedeutung hat wie $R_1$ und zusätzlich Fluor, Chlor oder Brom bedeutet; oder $R_1$ und $R_2$ gemeinsam für eine substituierte oder unsubstituierte $C_3$-$C_4$-Alkylenbrücke stehen; Y CN oder $COOR_3$ ist, worin $R_3$ Alkyl, Cycloalkyl, Aryl oder Aralkyl darstellt, und $R_4$ und $R_5$ einzeln je Alkyl, Cycloalkyl, Aralkyl oder Aryl oder zusammen Alkylen oder Oxaalkylen bedeuten.

FO 7.5/HH/sm*